# EUROPEAN PATENT APPLICATION

(11) **EP 2 103 618 A1**
(43) Date of publication of application: **23.09.2009**
(21) Application number: 08005327.5
(22) Date of filing: 20.03.2008
(51) Int. Cl.: C07D 498/18, A61K 31/537, A61P 35/00

(54) **Novel ansamitocin derivatives**

(71) Applicant: Leibniz Universität Hannover, 30167 Hannover (DE); Helmholtz-Zentrum für Infektionsforschung GmbH, 38124 Braunschweig (DE); Georg-August-Universität Göttingen, 37073 Göttingen (DE)
(72) Inventor: Sasse, Florenz, 38124 Braunschweig (DE); Kirschning, Andreas, 38678 Clausthal-Zellerfeld (DE); Grond, Stephanie, 37077 Göttingen (DE)
(74) Representative: Forstmeyer, Dietmar

(57) **Abstract**

This invention relates to the field of biologically active compounds and specifically to novel ansamitocin derivatives, pharmaceutical compositions comprising these novel ansamitocin derivatives, methods for the production of the ansamitocin derivatives of the general formula (I): 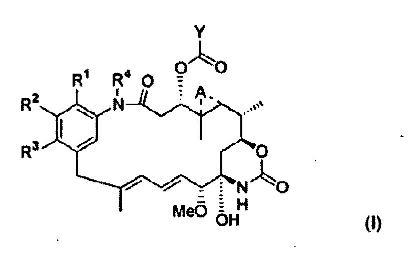 and their use for the treatment of cancer.

## Description

This invention relates to the field of biologically active compounds and specifically to novel ansamitocin derivatives, pharmaceutical compositions comprising these novel ansamitocin derivatives, methods for the production of the ansamitocin derivatives and their use for the treatment of cancer.

Ansamitocins are members of the ansamycin group of natural products (Rinehart K. L., Jr., Shield L. S., Fortschr. Chem. Org. Naturst., 1976, 33, 231-307) and are characterized by 19-member ansamacrolide structures attached to a chlorinated benzene ring chromophore. The ansamitocins have been obtained from bacterial sources, mainly *Actinosynnema pretiosum* (E. Higashide, M. Asai, K. Ootsu, S. Tanida, Y. Kozai, T. Hasegawa, T. Kishi, Y. Sugino, M. Yoneda, Nature 1977, 270, 721-722). Recently, the ansamitocin biosynthetic gene cluster for the biosynthesis of ansamitocins has been identified (WO 03/045312).

Ansamitocins are extraordinarily potent antitumor agents blocking the assembly of tubulin into functional microtubules (J. M. Cassady, K. K. Chan, H. G. Floss, E. Leistner, Chem. Pharm. Bull. 2004, 52, 1-26; Komoda Y., Kishi T., "Anticancer Agents Based on Natural Product Models", ed. by Douros J., Cassady J. M., Academic Press, New York, 1980, pp. 353-389; Reider P. J., Roland D. M., "The Alkaloids," Vol. 23, ed. by Brossi A., Academic Press, New York, 1984, pp. 71-156; Smith C. R., Jr., Powell R. G., "Alkaloids," Vol. 2, ed. by Pelletier S. W., John Wiley and Sons, New York, 1984, pp. 149-204).

In view of the fact that there hardly exist effective therapies for the treatment of cancer, there is a need for novel compounds having antitumor activity.

Therefore, the problem underlying the present invention is to provide novel compounds, namely ansamitocin derivatives having antitumor activity.

The present invention relates to an ansamitocin derivative of the general formula (I): 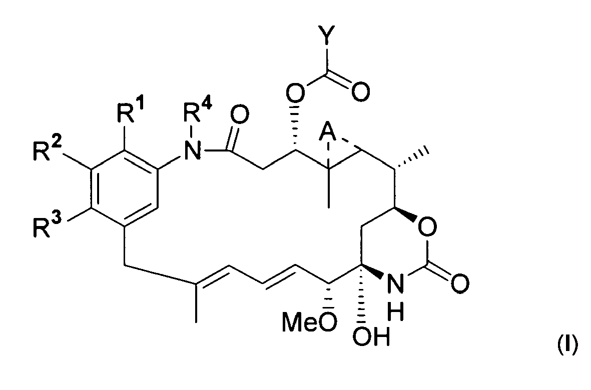 or a pharmacologically acceptable salt, solvate, hydrate or a pharmacologically acceptable formulation thereof, wherein
R¹ is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a hetero-alkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or R¹ is taken together with R² to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;
R² is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, or aryl;
R³ is a hydrogen atom or fluorine atom ;
R⁴ is a hydrogen atom or methyl group;
A represents a bond or an epoxy group;
Y is a methyl, isopropyl, or isobutyl group.

Compounds are generally described herein using standard nomenclature. For compounds having asymmetric centers, it should be understood that, unless otherwise specified, all of the optical isomers and mixtures thereof are encompassed. Compounds with two or more asymmetric elements can also be present as mixtures of diastereomers. In addition, compounds with carbon-carbon double bonds may occur in Z- and E- forms, with all isomeric forms of the compounds being included in the present invention unless otherwise specified. Where a compound exists in various tautomeric forms, a recited compound is not limited to any one specific tautomer, but rather is intended to encompass all tautomeric forms. Recited compounds are further intended to encompass compounds in which one or more atoms are replaced with an isotope, *i*.*e*., an atom having the same atomic number but a different mass number. By way of general example, and without limitation, isotopes of hydrogen include tritium and deuterium and isotopes of carbon include ¹¹C, ¹³C , and ¹⁴C.

Compounds according to the formulas provided herein, which have one or more stereogenic center(s), have an enantiomeric excess of at least 50%. For example, such compounds may have an enantiomeric excess of at least 60%, 70%, 80%, 85%, 90%, 95%, or 98%. Some embodiments of the compounds have an enantiomeric excess of at least 99%. It will be apparent that single enantiomers (optically active forms) can be obtained by asymmetric synthesis, synthesis from optically pure precursors or by resolution of the racemates. Resolution of the racemates can be accomplished, for example, by conventional methods such as crystallization in the presence of a resolving agent, or chromatography, using, for example, a chiral HPLC column.

Compounds herein may also be described using a general formula that includes variables such as, e.g., A, R¹-R⁴, Y, etc. Unless otherwise specified, each variable within such a formula is defined independently of any other variable, and any variable that occurs more than one time in a formula is defined independently at each occurrence. Thus, for example, if a group is shown to be substituted with 0-2 R^{*}, the group may be unsubstituted or substituted with up to two R^{*} groups and R^{*} at each occurrence is selected independently from the definition of R^{*}. Also, combinations of substituents and/or variables are permissible only if such combinations result in stable compounds, *i*.*e*., compounds that can be isolated, characterized and tested for biological activity.

An "ansamitocin derivative" disclosed herein is a compound that shares the 19-member ansamacrolide ring structure with the naturally occuring ansamitocins P1, P2, P3, or P4 but is not identical to them. In other words, naturally occuring ansamitocins P1, P2, P3 and P4 are excluded.

A "pharmaceutically acceptable salt" of a compound disclosed herein preferably is an acid or base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids.

Suitable pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4, *i*.*e*., 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of nonaqueous media, such as ether, ethyl acetate, ethanol, isopropanol or acetonitrile, is preferred.

It will be apparent that each compound of formula (I) may, but need not, be present as a hydrate, solvate or non-covalent complex. In addition, the various crystal forms and polymorphs are within the scope of the present invention, as are prodrugs of the compounds of formula (I) provided herein.

A "prodrug" is a compound that may not fully satisfy the structural requirements of the compounds provided herein, but is modified *in vivo*, following administration to a subject or patient, to produce a compound of formula (I) provided herein. For example, a prodrug may be an acylated derivative of a compound as provided herein. Prodrugs include compounds wherein hydroxy, carboxy, amine or sulfhydryl groups are bonded to any group that, when administered to a mammalian subject, cleaves to form a free hydroxy, carboxy, amino, or sulfhydryl group, respectively. Examples of prodrugs include, but are not limited to, acetate, formate, phosphate and benzoate derivatives of alcohol and amine functional groups within the compounds provided herein. Prodrugs of the compounds provided herein may be prepared by modifying functional groups present in the compounds in such a way that the modifications are cleaved *in vivo* to generate the parent compounds.

A "substituent," as used herein, refers to a molecular moiety that is covalently bonded to an atom within a molecule of interest, e.g. to a compound of formula (I), (II), (III) or (IV) or a prodrug thereof. For example, a "ring substituent" may be a moiety such as a halogen, alkyl group, haloalkyl group or other substituent described herein that is covalently bonded to an atom, preferably a carbon or nitrogen atom, that is a ring member. The term "substituted," as used herein, means that any one or more hydrogens on the designated atom is replaced with a selection from the indicated substituents, provided that the designated atom's normal valence is not exceeded, and that the substitution results in a stable compound, *i*.*e*., a compound that can be isolated, characterized and tested for biological activity. When a substituent is oxo, *i*.*e*., =O, then 2 hydrogens on the atom are replaced. An oxo group that is a substituent of an aromatic carbon atom results in a conversion of -CH- to -C(=O)- and a loss of aromaticity. For example a pyridyl group substituted by oxo is a pyridone.

The expression alkyl preferably refers to a saturated, straight-chain or branched hydrocarbon group that contains from 1 to 20 carbon atoms, preferably from 1 to 12 carbon atoms, more preferably from 1 to 6 carbon atoms, for example a methyl, ethyl, propyl, iso-propyl, n-butyl, iso-butyl, *sec*-butyl, *tert*-butyl, n-pentyl, n-hexyl, 2,2-dimethylbutyl or n-octyl group.

The expressions alkenyl and alkynyl refer to at least partially unsaturated, straight-chain or branched hydrocarbon groups that contain from 2 to 20 carbon atoms, preferably from 2 to 12 carbon atoms, more preferably from 2 to 6 carbon atoms, for example an ethenyl, allyl, acetylenyl, propargyl, isoprenyl or hex-2-enyl group. Preferably, alkenyl groups have one or two, more preferably one, double bond(s) and alkynyl groups have one or two, more preferably one, triple bond(s).

Furthermore, the terms alkyl, alkenyl and alkynyl refer to groups in which one or more hydrogen atoms have been replaced each independently of the others by a halogen atom, preferably F or Cl, such as, for example, a 2,2,2-trichloroethyl or a trifluoromethyl group.

The expression heteroalkyl preferably refers to an alkyl, alkenyl or alkynyl group, for example heteroalkenyl, heteroalkynyl, in which one or more, preferably 1, 2 or 3 carbon atoms have been replaced each independently of the others by an oxygen, nitrogen, phosphorus, boron, selenium, silicon or sulphur atom, preferably oxygen, sulphur or nitrogen. The expression heteroalkyl, for example, refers to an alkoxy group. Furthermore, heteroalkyl preferably refers to a carboxylic acid or to a group derived from a carboxylic acid such as, for example, acyl, acylalkyl, alkoxycarbonyl, acyloxy, acyloxyalkyl, carboxyalkylamide, alkylcarbamoylalkyl, alkylcarbamoyloxyalkyl, alkylureidoalkyl, or alkoxycarbonyloxy.

Examples of heteroalkyl groups are groups of formulas -S-Y^{a}-L, -S-Y^{a}-CO-NR^{a}R^{b}, -Y^{a}-NR^{c}-CO-NR^{a}R^{b}, -Y^{a}-NR^{c}-CO-O-R^{d}, -Y^{a}-NR^{c}-CO-R^{d}, -Y^{a}-NR^{c}-CO-NR^{d}-L, -Y^{a}-NR^{c}-CS-NR^{d}-L, -Y^{a}-O-CO-NR^{a}R^{b}, -Y^{a}-CO-NR^{a}R^{b}, -O-Y^{a}-CO-NR^{a}R^{b}, -Y^{a}-NR^{c}-CO-L, , -Y^{a}-O-CO-O-R^{c}, -Y^{a}-O-CO-R^{c}, -Y^{a}-O-R^{c}, -Y^{a}-CO-L, -Y^{a}-NR^{a}R^{b}, R^{c}-S-Y^{a}-, R^{a}-N(R^{b})-Y^{a}-, R^{c}-CO-Y^{a}-, R^{c}-O-CO-Y^{a}-, R^{c}-CO-O-Y^{a}-, R^{c}-CO-N(R^{b})-Y^{a}-, R^{a}-N(R^{b})-CO-Y^{a}-, R^{c}-SO-Y^{a}-, R^{c}-SO₂-Y^{a}-, -Y^{a}-NR^{c}-SO₂-NR^{a}R^{b}, -Y^{a}-SO₂-NR^{a}R^{b}, -Y^{a}-NR^{c}-SO₂-R^{d}, R^{a}-O-CO-N(R^{b})-Y^{a}-, R^{a}-N(R^{b})-C(=NR^{d})-N(R^{c})-Y^{a}-, R^{c}-S-CO-Y^{a}-, R^{c}-CO-S-Y^{a}-, R^{c}-S-CO-N(R^{b})-Y^{a}-, R^{a}-N(R^{b})-CO-S-Y^{a}-, R^{c}-S-CO-O-Y^{a}-, R^{c}-O-CO-S-Y^{a}-, R^{c}-S-CO-S-Y^{a}-; wherein R^{a} being a hydrogen atom, a C₁-C₆alkyl, a C₂-C₆alkenyl, a C₂-C₆alkynyl , or is joined to R^{b} to form a 4- to 10-membered cycloalkyl or heterocycloalkyl; R^{b} being a hydrogen atom, a C₁-C₆alkyl, a C₂-C₆alkenyl or a C₂-C₆alkynyl, or taken together with R^{a} to form a 4- to 10-membered cycloalkyl or heterocycloalkyl; R^{c} being a hydrogen atom, an optionally substituted C₁-C₈alkyl, an optionally substituted C₂-C₈alkenyl or an optionally substituted C₂-C₈alkynyl ; R^{d} being a hydrogen atom, optionally substituted C₁-C₈alkyl, optionally substituted C₂-C₈alkenyl or optionally substituted C₂-C₈alkynyl; L being a cycloalkyl, heterocycloalkyl, alkylcycloalkyl, heteroalkylcycloalkyl, optionally substituted aryl, optionally substituted heteroaryl, aralkyl, or heteroaralkyl; and Y^{a} being a bond, a C₁-C₆alkylene, a C₂-C₆alkenylene or a C₂-C₆alkynylene group; each heteroalkyl group containing at least one carbon atom and it being possible for one or more hydrogen atoms to have been replaced by fluorine or chlorine atoms. Specific examples of heteroalkyl groups are methoxy, trifluoromethoxy, ethoxy, n-propyloxy, isopropyloxy, *tert*-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, enol ether, dimethylaminomethyl, dimethylaminoethyl, acetyl, propionyl, butyryloxy, acetyloxy, methoxycarbonyl, ethoxycarbonyl, isobutyrylaminomethyl, N-ethyl-N-methylcarbamoyl and N-methylcarbamoyl. Further examples of heteroalkyl groups are nitrile, isonitrile, cyanate, thiocyanate, isocyanate, isothiocyanate and alkylnitrile groups. An example of a heteroalkylene group is a group of formulas -CH₂CH(OH)- or - CONH-.

The expression cycloalkyl preferably refers to a saturated or partially unsaturated cyclic group that contains one or more rings, preferably 1 or 2, containing from 3 to 14 ring carbon atoms, preferably from 3 to 10, more preferably 3, 4, 5, 6 or 7, ring carbon atoms. In an embodiment a partially unsaturated cyclic group has one, two or more double bonds, such as a cycloalkenyl group. The expression cycloalkyl preferably refers furthermore to groups in which one or more hydrogen atoms have been replaced each independently of the others by fluorine, chlorine, bromine or iodine atoms or by OH, =O, SH, =S, NH₂, =NH, CN or NO₂ groups, thus, for example, cyclic ketones such as, for example, cyclohexanone, 2-cyclohexenone or cyclopentanone. Further specific examples of a cycloalkyl group is a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetralin, cyclopentylcyclohexyl, fluorocyclohexyl or cyclohex-2-enyl group.

The expression heterocycloalkyl preferably refers to a cycloalkyl group as defined above in which one or more, preferably 1, 2 or 3, ring carbon atoms have been replaced each independently of the others by an oxygen, nitrogen, silicon, selenium, phosphorus or sulphur atom, preferably oxygen, sulphur or nitrogen. A heterocycloalkyl group has preferably 1 or 2 ring(s) containing from 3 to 10, more preferably 3, 4, 5, 6 or 7, ring atoms. The expression heterocycloalkyl preferably refers furthermore to groups in which one or more hydrogen atoms have been replaced each independently of the others by fluorine, chlorine, bromine or iodine atoms or by OH, =O, SH, =S, NH₂, =NH, CN or NO₂ groups. Examples are a piperidyl, piperazinyl, morpholinyl, urotropinyl, pyrrolidinyl, tetrahydrothiophenyl, tetrahydropyranyl, tetrahydrofuryl or 2-pyrazolinyl group and also a lactam, a lactone, a cyclic imide and a cyclic anhydride.

The expression alkylcycloalkyl preferably refers to a group containing both cycloalkyl and also an alkyl, alkenyl or alkynyl group in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two ring systems having from 3 to 10, preferably 3, 4, 5, 6 or 7, carbon atoms, and one or two alkyl, alkenyl or alkynyl groups having 1 or 2 to 6 carbon atoms, the cyclic groups being optionally substituted.

The expression heteroalkylcycloalkyl preferably refers to alkylcycloalkyl groups as defined above in which one or more, preferably 1, 2 or 3, carbon atoms have been replaced each independently of the others by an oxygen, nitrogen, silicon, selenium, phosphorus or sulphur atom, preferably oxygen, sulphur or nitrogen. A heteroalkylcycloalkyl group preferably contains 1 or 2 ring systems having from 3 to 10, preferably 3, 4, 5, 6 or 7, ring atoms, and one or two alkyl, alkenyl, alkynyl or heteroalkyl groups having from 1 or 2 to 6 carbon atoms. Examples of such groups are alkylheterocycloalkyl, heterocycloalkylalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being optionally substituted and saturated or mono-, di- or tri-unsaturated.

The expression aryl or Ar preferably refers to an aromatic group that contains one or more rings containing from 6 to 14 ring carbon atoms, preferably from 6 to 10, more preferably 6, ring carbon atoms. The expression aryl (or Ar) preferably refers furthermore to groups in which one or more hydrogen atoms have been replaced each independently of the others by fluorine, chlorine, bromine or iodine atoms or by OH, SH, NH₂, CN or NO₂ groups. Examples are a phenyl, naphthyl, biphenyl, 2-fluorophenyl, anilinyl, 3-nitrophenyl or 4-hydroxyphenyl group.

The expression heteroaryl preferably refers to an aromatic group that contains one or more rings containing from 5 to 14 ring atoms, preferably from 5 to 10, more preferably 5 or 6, ring atoms, and contains one or more, preferably 1, 2, 3 or 4, oxygen, nitrogen, phosphorus or sulphur ring atoms, preferably O, S or N. Furthermore, the expression heteroaryl preferably refers to groups in which one or more hydrogen atoms have been replaced each independently of the others by fluorine, chlorine, bromine or iodine atoms or by OH, =O, SH, NH₂, =NH, CN or NO₂ groups. Examples are 4-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl.

The expression aralkyl preferably refers to a group containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1 H-indene, tetralin, dihydronaphthalene, indanone, phenylcyclopentyl, cumene, cyclohexylphenyl, fluorene and indan. An aralkyl group preferably contains one or two aromatic ring systems, 1 or 2 rings, containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl preferably refers to an aralkyl group as defined above in which one or more, preferably 1, 2, 3 or 4, carbon atoms have been replaced each independently of the others by an oxygen, nitrogen, silicon, selenium, phosphorus, boron or sulphur atom, preferably oxygen, sulphur or nitrogen, that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems, 1 or 2 rings, containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced each independently of the others by oxygen, sulphur or nitrogen atoms.

Examples of heteroaralkyl groups are aryloxy, arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl, heteroalkylheteroarylalkyl and heteroarylheteroalkylheterocycloalkyl groups, the cyclic groups being saturated or mono-, di- or tri-unsaturated. Specific examples are a tetrahydro-isoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

The expressions cycloalkyl, heterocycloalkyl, alkylcycloalkyl, heteroalkylcycloalkyl, aryl, heteroaryl, aralkyl and heteroaralkyl also refer to groups in which one or more hydrogen atoms of such groups have been replaced each independently of the others by fluorine, chlorine, bromine or iodine atoms or by OH, =O, SH, =S, NH₂, =NH, CN or NO₂ groups.

The expression "substituted" as used in conncetion with any group especially refers to a group in which one or more hydrogen atoms have been replaced each independently of the others by fluorine, chlorine, bromine or iodine atoms or by OH, =O, SH, =S, NH₂, =NH, CN or NO₂ groups. This expression refers furthermore to a group in which one or more hydrogen atoms have been replaced each independently of the others by an unsubstituted C₁-C₆alkyl, unsubstituted C₂-C₆alkenyl, unsubstituted C₂-C₆alkynyl, unsubstituted C₁-C₆heteroalkyl, unsubstituted C₃-C₁₀cycloalkyl, unsubstituted C₂-C₉heterocycloalkyl, unsubstituted C₆-C₁₀aryl, unsubstituted C₁-C₉heteroaryl, unsubstituted C₇-C₁₂aralkyl or unsubstituted C₂-C₁₁heteroaralkyl group.

The expression "halogen" or "halogen atom" as preferably used herein means fluorine, chlorine, bromine, iodine.

As used herein a wording defining the limits of a range of length such as, e. g., "from 1 to 5" means any integer from 1 to 5, i. e. 1, 2, 3, 4 and 5. In other words, any range defined by two integers explicitly mentioned is meant to comprise and disclose any integer defining said limits and any integer comprised in said range.

The present invention preferably relates to an ansamitocin derivative according to formula (I): 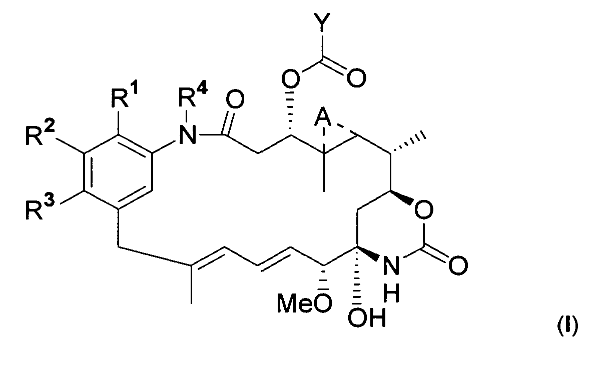 or a pharmacologically acceptable salt, solvate, hydrate or a pharmacologically acceptable formulation thereof, wherein
R¹ is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, hetero-alkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or
R¹ is taken together with R² to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;
R² is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, or aryl;
R³ is a hydrogen atom or fluorine atom ;
R⁴ is a hydrogen atom or methyl group;
A represents a bond or an epoxy group;
Y is a methyl, isopropyl, or isobutyl group; provided that
(i) the naturally occuring ansamitocines P1, P2, P3 and P4 are excluded; and/or
(ii) R¹ is not fluorine, chlorine or bromine atom if R² and R³ are hydrogen atoms, R⁴ is methyl, A is epoxy, and Y is isopropyl; and/or
(iii) R¹ is not hydrogen atom if R² is methoxy, R³ is hydrogen atom, R⁴ is methyl, A is epoxy, and Y is isopropyl.

Preferred are ansamitocin derivatives - in the following also simply referred to as compounds - of formula (I), wherein R¹ is a hydrogen atom, halogen atom, hydroxy, C1-C6 alkyl, C2-C6 alkenyl; C2-C6 alkynyl, C2-C6 heteroalkyl, alkoxycarbonyl, aryl, aralkyl, wherein alkyl, alkenyl, alkynyl, heteroalkyl, alkoxycarbonyl, aryl and aralkyl can be substituted with from 1 to 3 substituents which substituents are each independently selected from halogen atom, hydroxy, nitro, amino, alkoxy, carboxyl, alkynyl, alkenyl, aryl, sulfonyl, phosphoryl, anticalins, antibodies, folic acid, fluorescence dyes or labels, or molecular probes, or
R¹ is taken together with R² to form a 5- to 8-membered carbocyclic or heterocyclic ring that is substituted with from 0 to 3 substituents which substituents are each independently selected from halogen atom, hydroxy, nitro, amino, alkoxy, carboxyl, alkynyl, alkenyl, or aryl.

Further preferred are compounds of formula (I) wherein R1 is (a) a hydrogen atom, halogen atom or hydroxy, or (b) alkoxycarbonyl, aryl, vinyl, allyl, benzyl, ethynyl, or propargyl each of which can be substituted with from 1 to 2 substituents independently selected from halogen atom, hydroxy, amino, carboxyl, alkoxy, alkyl, or aryl; or R¹ is taken together with R² to form a heterocycloalkyl.

Especially preferred are compounds of formula (I) wherein R¹ is a hydrogen atom, halogen atom or hydroxy; or R¹ is taken together with R² to form a 1,3 dioxolen.

Further preferred are compounds of formula (I) wherein R² is a hydrogen atom, halogen atom, cyano, methoxy, trifluoromethyl, propadienyl, ethynyl, propargyl.

Further preferred are compounds of formula (I) wherein R³ is a hydrogen atom.

Further preferred are compounds of formula (I) wherein R² is a hydrogen atom or methoxy; and
R³ is a hydrogen atom.

Further preferred are compounds of formula (I) wherein R¹ is a hydrogen atom;
R² is a halogen atom, cyano, trifluoromethyl, propadienyl, ethynyl, propargyl; and
R³ is a hydrogen atom.

Further preferred are compounds of formula (I) wherein R⁴ is a methyl group.

Further preferred are compounds of formula (I) wherein Y is an isopropyl group.

Further preferred are compounds of formula (I) wherein A is an epoxy group.

It is to be noted that the present invention also encompasses all possible combinations of all preferred embodiments.

Especially preferred, the compound according to formula (I) is a compound according to formula (II): 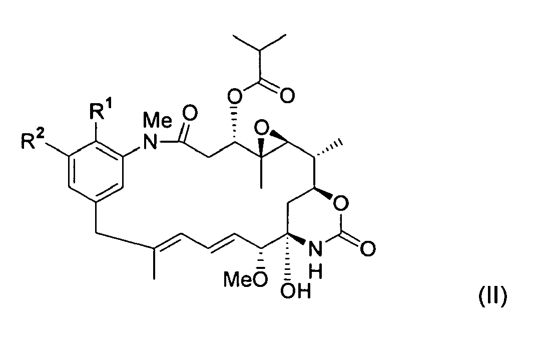 wherein
R¹ is a hydrogen, fluorine, chlorine, bromine, iodine or hydroxy; and
R² is a hydrogen atom or methoxy.

According to a further embodiment, compounds of formula (II) are excluded, wherein
(i) R¹ is fluorine, chlorine or bromine and R² is hydrogen;
(ii) R¹ is hydrogen and R² is methoxy.

Further, especially preferred, the compound according to formula (I) is a compound according to formula (III): 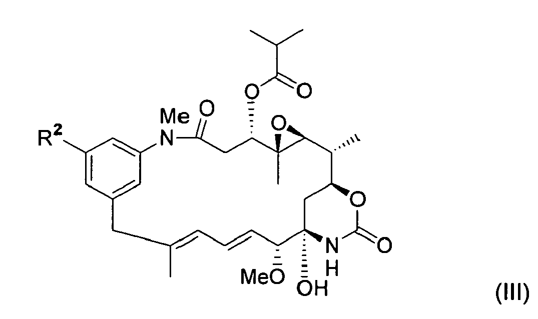 wherein
R² is fluorine, bromine, cyano, trifluoromethyl, propadienyl, ethynyl, or propargyl.

Further, especially preferred, the compound according to formula (I) is a compound according to formula (IV):

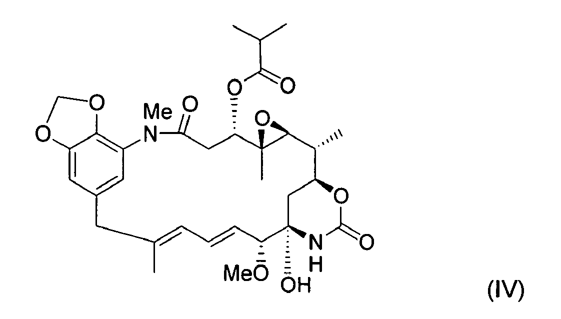

The compounds of formula (I), (II), (III) or (IV) according to the present invention each have improved properties, especially, low toxicity, low drug drug interaction, improved bioavailability especially with regard to oral administration, improved metabolic stability, and improved solubility.

Ansamitocine derivatives provided herein exhibit high antitumor activity on cultured human tumor cell lines, i.e. an antiproliferative activity with an inhibition constant (IC₅₀) of less than 5 ng/ml.

The activity and more specifically the pharmacological activity of the ansamitocine derivatives according to the present invention can be assessed using appropriate *in vitro* assays. For instance, the IC₅₀ values of the antitumor compounds according to the present invention may be determined via a depletion assay of cell growth. Preferred compounds of the invention have an IC₅₀ (half-maximal inhibitory concentration) of about 5 ng/ml or less, still more preferably an IC₅₀ of about 1 ng/ml or less, or even 0,5 ng/ml or less, even more preferably an IC₅₀ of about 0,1 ng/ml or less, or even 10 pg/ml or less in the assays mentioned above.

The therapeutic use of compounds of formula (I), (II), (III) or (IV), their pharmacologically acceptable salts, prodrugs, solvates and hydrates and also formulations and pharmaceutical compositions containing the same are within the scope of the present invention. The present invention also relates to the use of those compounds etc. of formula (I), (II), (III) or (IV) as active ingredients in the preparation or manufacture of a medicament, especially, the use of compounds of formula (I), (II), (III) or (IV), their pharmacologically acceptable salts, prodrugs or solvates and hydrates and also formulations and pharmaceutical compositions for the treatment of cancer as well as their use for the preparation of medicaments for the treatment of cancer.

The pharmaceutical compositions according to the present invention comprise at least one compound of formula (I), (II), (III) or (IV) and, optionally, one or more carrier substances, excipients and/or adjuvants. Pharmaceutical compositions may additionally comprise, for example, one or more of water, buffers such as, *e.g.*, neutral buffered saline or phosphate buffered saline, ethanol, mineral oil, vegetable oil, dimethylsulfoxide, carbohydrates such as *e.g.*, glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical compositions provided herein. For instance, the compounds of the invention may advantageously be employed in combination with an antibiotic, anti-fungal, or anti-viral agent, an-anti histamine, a non-steroidal anti-inflammatory drug, a disease modifying anti-rheumatic drug, a cytostatic drug, a drug with smooth muscle activity modulatory activity or mixtures of the aforementioned.

Pharmaceutical compositions may be formulated for any appropriate route of administration, including, for example, topical such as, *e.g.*, transdermal or ocular, oral, buccal, nasal, vaginal, rectal or parenteral administration. The term parenteral as used herein includes subcutaneous, intradermal, intravascular such as, *e.g.*, intravenous, intramuscular, spinal, intracranial, intrathecal, intraocular, periocular, intraorbital, intrasynovial and intraperitoneal injection, as well as any similar injection or infusion technique. In certain embodiments, compositions in a form suitable for oral use are preferred. Such forms include, for example, tablets, troches, lozenges, aqueous or oily suspensions, dispersible powders or granules, emulsion, hard or soft capsules, or syrups or elixirs. Within yet other embodiments, compositions provided herein may be formulated as a lyophilizate. Formulation for topical administration may be preferred for certain conditions such as, *e.g.*, in the treatment of skin conditions such as burns or itch.

Compositions intended for oral use may further comprise one or more components such as sweetening agents, flavoring agents, coloring agents and/or preserving agents in order to provide appealing and palatable preparations. Tablets contain the active ingredient in admixture with physiologically acceptable excipients that are suitable for the manufacture of tablets. Such excipients include, for example, inert diluents such as, *e.g.*, calcium carbonate, sodium carbonate, lactose, calcium phosphate or sodium phosphate, granulating and disintegrating agents such as, *e.g.*, corn starch or alginic acid, binding agents such as, *e.g.*, starch, gelatin or acacia, and lubricating agents such as, *e.g.*, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatin capsules wherein the active ingredient is mixed with an inert solid diluent such as, *e.g.*, calcium carbonate, calcium phosphate or kaolin, or as soft gelatin capsules wherein the active ingredient is mixed with water or an oil medium such as *e.g.*, peanut oil, liquid paraffin or olive oil.

Aqueous suspensions contain the active ingredient(s) in admixture with excipients suitable for the manufacture of aqueous suspensions. Such excipients include suspending agents suh as, *e.g.*, sodium carboxymethylcellulose, methylcellulose, hydropropylmethylcellulose, sodium alginate, polyvinylpyrrolidone, gum tragacanth and gum acacia; and dispersing or wetting agents such as, *e.g.*, naturally-occurring phosphatides such as lecithin, condensation products of an alkylene oxide with fatty acids such as polyoxyethylene stearate, condensation products of ethylene oxide with long chain aliphatic alcohols such as heptadecaethyleneoxycetanol, condensation products of ethylene oxide with partial esters derived from fatty acids and a hexitol such as polyoxyethylene sorbitol monooleate, or condensation products of ethylene oxide with partial esters derived from fatty acids and hexitol anhydrides such as polyethylene sorbitan monooleate. Aqueous suspensions may also comprise one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the active ingredients in a vegetable oil such as, *e.g.*, arachis oil, olive oil, sesame oil or coconut oil, or in a mineral oil such as liquid paraffin. The oily suspensions may contain a thickening agent such as beeswax, hard paraffin or cetyl alcohol. Sweetening agents such as those set forth above, and/or flavoring agents may be added to provide palatable oral preparations. Such suspensions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, such as sweetening, flavoring and coloring agents, may also be present.

Pharmaceutical compositions may also be in the form of oil-in-water emulsions. The oily phase may be a vegetable oil such as, *e.g.*, olive oil or arachis oil, a mineral oil such as, *e.g.*, liquid paraffin, or a mixture thereof. Suitable emulsifying agents include naturally-occurring gums such as, *e.g.*, gum acacia or gum tragacanth, naturally-occurring phosphatides such as, *e.g*., soy bean lecithin, and esters or partial esters derived from fatty acids and hexitol, anhydrides such as, *e.g.*, sorbitan monoleate, and condensation products of partial esters derived from fatty acids and hexitol with ethylene oxide such as, *e.g.*, polyoxyethylene sorbitan monoleate. An emulsion may also comprise one or more sweetening and/or flavoring agents.

Syrups and elixirs may be formulated with sweetening agents, such as glycerol, propylene glycol, sorbitol or sucrose. Such formulations may also comprise one or more demulcents, preservatives, flavoring agents and/or coloring agents.

Compounds may be formulated for local or topical administration, such as for topical application to the skin or mucous membranes, such as in the eye. Formulations for topical administration typically comprise a topical vehicle combined with active agent(s), with or without additional optional components. Suitable topical vehicles and additional components are well known in the art, and it will be apparent that the choice of a vehicle will depend on the particular physical form and mode of delivery. Topical vehicles include water; organic solvents such as alcohols such as, *e.g.*, ethanol or isopropyl alcohol or glycerin; glycols such as, *e.g.*, butylene, isoprene or propylene glycol; aliphatic alcohols such as, *e.g.*, lanolin; mixtures of water and organic solvents and mixtures of organic solvents such as alcohol and glycerin; lipid-based materials such as fatty acids, acylglycerols including oils, such as, *e.g.*, mineral oil, and fats of natural or synthetic origin, phosphoglycerides, sphingolipids and waxes; protein-based materials such as collagen and gelatin; silicone-based materials, both non-volatile and volatile; and hydrocarbon-based materials such as microsponges and polymer matrices. A composition may further include one or more components adapted to improve the stability or effectiveness of the applied formulation, such as stabilizing agents, suspending agents, emulsifying agents, viscosity adjusters, gelling agents, preservatives, antioxidants, skin penetration enhancers, moisturizers and sustained release materials. Examples of such components are described in Martindale--The Extra Pharmacopoeia (Pharmaceutical Press, London 1993) and Martin (ed.), Remington's Pharmaceutical Sciences. Formulations may comprise microcapsules, such as hydroxymethylcellulose or gelatin-microcapsules, liposomes, albumin microspheres, microemulsions, nanoparticles or nanocapsules.

A topical formulation may be prepared in a variety of physical forms including, for example, solids, pastes, creams, foams, lotions, gels, powders, aqueous liquids, emulsions, sprays and skin patches. The physical appearance and viscosity of such forms can be governed by the presence and amount of emulsifier(s) and viscosity adjuster(s) present in the formulation. Solids are generally firm and non-pourable and commonly are formulated as bars or sticks, or in particulate form; solids can be opaque or transparent, and optionally can contain solvents, emulsifiers, moisturizers, emollients, fragrances, dyes/colorants, preservatives and other active ingredients that increase or enhance the efficacy of the final product. Creams and lotions are often similar to one another, differing mainly in their viscosity; both lotions and creams may be opaque, translucent or clear and often contain emulsifiers, solvents, and viscosity adjusting agents, as well as moisturizers, emollients, fragrances, dyes/colorants, preservatives and other active ingredients that increase or enhance the efficacy of the final product. Gels can be prepared with a range of viscosities, from thick or high viscosity to thin or low viscosity. These formulations, like those of lotions and creams, may also contain solvents, emulsifiers, moisturizers, emollients, fragrances, dyes/colorants, preservatives and other active ingredients that increase or enhance the efficacy of the final product. Liquids are thinner than creams, lotions, or gels and often do not contain emulsifiers. Liquid topical products often contain solvents, emulsifiers, moisturizers, emollients, fragrances, dyes/colorants, preservatives and other active ingredients that increase or enhance the efficacy of the final product.

Suitable emulsifiers for use in topical formulations include, but are not limited to, ionic emulsifiers, cetearyl alcohol, non-ionic emulsifiers like polyoxyethylene oleyl ether, PEG-40 stearate, ceteareth-12, ceteareth-20, ceteareth-30, ceteareth alcohol, PEG-100 stearate and glyceryl stearate. Suitable viscosity adjusting agents include, but are not limited to, protective colloids or non-ionic gums such as hydroxyethylcellulose, xanthan gum, magnesium aluminum silicate, silica, microcrystalline wax, beeswax, paraffin, and cetyl palmitate. A gel composition may be formed by the addition of a gelling agent such as chitosan, methyl cellulose, ethyl cellulose, polyvinyl alcohol, polyquaterniums, hydroxyethylcellulose, hydroxypropylcellulose, hydroxypropylmethylcellulose, carbomer or ammoniated glycyrrhizinate. Suitable surfactants include, but are not limited to, nonionic, amphoteric, ionic and anionic surfactants. For example, one or more of dimethicone copolyol, polysorbate 20, polysorbate 40, polysorbate 60, polysorbate 80, lauramide DEA, cocamide DEA, and cocamide MEA, oleyl betaine, cocamidopropyl phosphatidyl PG-dimonium chloride, and ammonium laureth sulfate may be used within topical formulations.

Suitable preservatives include, but are not limited to, antimicrobials such as methylparaben, propylparaben, sorbic acid, benzoic acid, and formaldehyde, as well as physical stabilizers and antioxidants such as vitamin E, sodium ascorbate/ascorbic acid and propyl gallate. Suitable moisturizers include, but are not limited to, lactic acid and other hydroxy acids and their salts, glycerin, propylene glycol, and butylene glycol. Suitable emollients include lanolin alcohol, lanolin, lanolin derivatives, cholesterol, petrolatum, isostearyl neopentanoate and mineral oils. Suitable fragrances and colors include, but are not limited to, FD&C Red No. 40 and FD&C Yellow No. 5. Other suitable additional ingredients that may be included in a topical formulation include, but are not limited to, abrasives, absorbents, anti-caking agents, anti-foaming agents, anti-static agents, astringents such as, *e.g.*, witch hazel, alcohol and herbal extracts such as chamomile extract, binders/excipients, buffering agents, chelating agents, film forming agents, conditioning agents, propellants, opacifying agents, pH adjusters and protectants.

An example of a suitable topical vehicle for formulation of a gel is: hydroxypropylcellulose (2.1%); 70/30 isopropyl alcohol/water (90.9%); propylene glycol (5.1%); and Polysorbate 80 (1.9%). An example of a suitable topical vehicle for formulation as a foam is: cetyl alcohol (1.1%); stearyl alcohol (0.5%); Quaternium 52 (1.0%); propylene glycol (2.0%); Ethanol 95 PGF3 (61.05%); deionized water (30.05%); P75 hydrocarbon propellant (4.30%). All percents are by weight.

Typical modes of delivery for topical compositions include application using the fingers; application using a physical applicator such as a cloth, tissue, swab, stick or brush; spraying including mist, aerosol or foam spraying; dropper application; sprinkling; soaking; and rinsing. Controlled release vehicles can also be used, and compositions may be formulated for transdermal administration as a transdermal patch.

A pharmaceutical composition may be formulated as inhaled formulations, including sprays, mists, or aerosols. Such formulations are particularly useful for the treatment of asthma or other respiratory conditions. For inhalation formulations, the compounds provided herein may be delivered via any inhalation methods known to those skilled in the art. Such inhalation methods and devices include, but are not limited to, metered dose inhalers with propellants such as CFC or HFA or propellants that are physiologically and environmentally acceptable. Other suitable devices are breath operated inhalers, multidose dry powder inhalers and aerosol nebulizers. Aerosol formulations for use in the subject method typically include propellants, surfactants and co-solvents and may be filled into conventional aerosol containers that are closed by a suitable metering valve.

Inhalant compositions may comprise liquid or powdered compositions containing the active ingredient that are suitable for nebulization and intrabronchial use, or aerosol compositions administered via an aerosol unit dispensing metered doses. Suitable liquid compositions comprise the active ingredient in an aqueous, pharmaceutically acceptable inhalant solvent, *e.g.*, isotonic saline or bacteriostatic water. The solutions are administered by means of a pump or squeeze-actuated nebulized spray dispenser, or by any other conventional means for causing or enabling the requisite dosage amount of the liquid composition to be inhaled into the patient's lungs. Suitable formulations, wherein the carrier is a liquid, for administration, as for example, a nasal spray or as nasal drops, include aqueous or oily solutions of the active ingredient.

Formulations or compositions suitable for nasal administration, wherein the carrier is a solid, include a coarse powder having a particle size, for example, in the range of 20 to 500 microns which is administered in the manner in which snuff is administered, *i*.*e*., by rapid inhalation through the nasal passage from a container of the powder held close up to the nose. Suitable powder compositions include, by way of illustration, powdered preparations of the active ingredient thoroughly intermixed with lactose or other inert powders acceptable for intrabronchial administration. The powder compositions can be administered via an aerosol dispenser or encased in a breakable capsule which may be inserted by the patient into a device that punctures the capsule and blows the powder out in a steady stream suitable for inhalation.

Pharmaceutical compositions may also be prepared in the form of suppositories such as *e.g.*, for rectal administration. Such compositions can be prepared by mixing the drug with a suitable non-irritating excipient that is solid at ordinary temperatures but liquid at the rectal temperature and will therefore melt in the rectum to release the drug. Suitable excipients include, for example, cocoa butter and polyethylene glycols.

Pharmaceutical compositions may be formulated as sustained release formulations such as, *i.e.,* a formulation such as a capsule that creates a slow release of modulator following administration. Such formulations may generally be prepared using well known technology and administered by, for example, oral, rectal or subcutaneous implantation, or by implantation at the desired target site. Carriers for use within such formulations are biocompatible, and may also be biodegradable; preferably the formulation provides a relatively constant level of modulator release. The amount of modulator contained within a sustained release formulation depends upon, for example, the site of implantation, the rate and expected duration of release and the nature of the condition to be treated or prevented.

For the treatment of cancer, the dose of the biologically active compound according to the invention may vary within wide limits and may be adjusted to individual requirements. Active compounds according to the present invention are generally administered in a therapeutically effective amount. Preferred doses range from about 0.1 mg to about 140 mg per kilogram of body weight per day, about 0.5 mg to about 7 g per patient per day. The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

It will be understood, however, that the specific dose level for any particular patient will depend upon a variety of factors including the activity of the specific compound employed, the age, body weight, general health, sex, diet, time of administration, route of administration, and rate of excretion, drug combination, *i*.*e*. other drugs being used to treat the patient, and the severity of the particular disease undergoing therapy.

Preferred compounds of the invention will have certain pharmacological properties. Such properties include, but are not limited to oral bioavailability, such that the preferred oral dosage forms discussed above can provide therapeutically effective levels of the compound *in vivo*.

Ansamitocin derivatives provided herein are preferably administered to a patient such as, *e.g.*, a human, orally or topically, and are present within at least one body fluid or tissue of the patient. Accordingly, the present invention further provides methods for treating patients suffering from cancer. As used herein, the term "treatment" encompasses both disease-modifying treatment and symptomatic treatment, either of which may be prophylactic, *i*.*e*., before the onset of symptoms, in order to prevent, delay or reduce the severity of symptoms, or therapeutic, *i*.*e*., after the onset of symptoms, in order to reduce the severity and/or duration of symptoms. Patients may include but are not limited to primates, especially humans, domesticated companion animals such as dogs, cats, horses, and livestock such as cattle, pigs, sheep, with dosages as described herein.

It is also within the present invention that the compounds according to the invention are used as or for the manufacture of a diagnostic agent, whereby such diagnostic agent is for the diagnosis of the diseases and conditions which can be addressed by the compounds of the present invention for therapeutic purposes as disclosed herein.

For various applications, the compounds of the invention can be labelled by isotopes, fluorescence or luminescence markers, antibodies or antibody fragments, any other affinity label like nanobodies, aptamers, peptides etc., enzymes or enzyme substrates. These labelled compounds of this invention are useful for mapping the location of BK receptors *in vivo, ex vivo, in vitro* and *in situ* such as, *e.g*. in tissue sections via autoradiography and as radiotracers for positron emission tomography (PET) imaging, single photon emission computerized tomography (SPECT) and the like to characterize those receptors in living subjects or other materials. The labelled compounds according to the present invention may be used in therapy, diagnosis and other applications such as research tools *in vivo* and *in vitro*, in particular the applications disclosed herein.

Also the following methods for producing compounds of formula (I), (II), (III) or (IV) (ansamitocine derivatives) lie within the scope of the present invention.

Compounds of formula (I), (II), (III) or (IV) (ansamitocine derivatives) can be produced by culturing a bacterium of the genus *Actinosynnema pretiosum.* It is understood that the production of ansamitocine derivatives is not limited to the use of the particular organism described herein, which is given for illustrative purpose only. The invention also includes the use of any mutants which are capable of producing ansamitocine derivatives including natural mutants as well as artificial mutants, e.g. genetically manipulated mutants and the expression of the gene cluster responsible for biosynthesis in a producer strain or by heterologous expression in host strains.

Compounds of formula (I), (II), (III) or (IV) can be attained by alteration of the growth conditions of *Actinosynnema pretiosum* strains, by mutasynthesis, or by chemically reacting ansamitocin derivatives.

A microorganism, which can be used for the production of ansamitocin derivatives is the 3-amino-5-hydroxybenzoic acid (AHBA) blocked mutant HGF073 of the ansamitocin producer *Actinosynnema pretiosum.* Mutant HGF073 (T.-W. Yu, L. Bai, D. Clade, D. Hoffmann, S. Toelzer, K. Q. Trinh, J. Xu, S. J. Moss, E. Leistner, H. G. Floss, Proc. Nat Acad. Sci. USA 2002, 99, 7968 - 7973) has also been deposited at the German Collection of Microorganisms and Cell Cultures (DSMZ) Braunschweig, Germany.

Ansamitocins are produced in liquid culture, by growing in media containing one or several different carbon sources, and one or different nitrogen sources. Also salts are essential for growth and production. Suitable carbon sources are different mono-, di-, and polysaccharides like glucose, sucrose, lactose or carbon from amino acids like peptones. Nitrogen sources are ammonium, nitrate, urea, chitin or nitrogen from amino acids.

The following inorganic ions support the growth or are essential in synthetic media: Mg-ions, Ca-ions, Fe-ions, Mn-ions, Zn-ions, K-ions, sulfate-ions, Cl-ions, phosphate-ions.

Temperatures for growth and production are between 10°C to 40 °C, preferred temperatures are between 15°C and 37 °C, especially between 27°C to 33°C.

### Production of Ansamitocin derivatives by altering the growth conditions

The producing microorganism creates the ansamitocins by biosynthetic machinery generating many bioactive agents. The main natural product of the biosynthesis is ansamitocin P3. This product can be generated by Mutant HGF073 using the following growth conditions.

Cells harboring mutant HGF073 are grown on a YMG agar plate for 3 days at 30°C. For the preparation of a preculture 50 ml YMG medium (4 g/L Yeast extract, 10 g/L malt extract, 4 g/L Glucose, destilled water, pH 7.3 ) contained in an 300 ml Erlenmeyer flask is inoculated with a three day old single colony from the agar plate. Precultures are incubated at 30°C, 225 rpm for 2 days. Fermentation for ansamitocin production is carried out in medium K (60 g/L Dextrin from potato starch, 5.25 g/L Proflo cotton seed starch, 0.3 g/L K₂HPO₄, 30 g/L maltose, 4.5 g/L yeast extract, 2 mg/L FeSO₄x7 H₂O, 5 g/L CaCO₃, destilled water, pH 7.2, 3 mL sterile filtered 3% L-valine solution (per 25 mL culture broth) supplemented with AHBA, wherein fermantation is started by inoculating the cultures with 2 ml of the preculture per 25 ml culture broth. Incubation for 7 days at 30 °C in a rotary incubator at 225 rpm. After 7 days the cultures are harvested and two times extracted with ethyl acetate (20 ml). The ethyl acetate portions are concentrated in vacuo and the residue is dissolved in methanol (1 ml). The pure product can be isolated by applying HPLC-purification procedures (column 250x8 mm (LiCHrospher RP Select B), 10 µm, eluent: methanol/H₂O, gradient 20% methanol to 100% methanol in 30 min, 5 min 100% methanol, flow: 6 ml/min; UV detection at 254 nm).

By altering the fermentation conditions, for example, supplementing the culture medium with additional salts like NaBr, novel ansamitocin derivatives can be obtained.

### Example 1: Production of Br-AP-2, Br-AP-3 and Br-AP-4

Mutant HGF073 was cultured as described above, except that the medium K was additionally supplemented with NaBr (0,1 mol/L). The cultures were harvested after 7 days, extracted with ethyl acetate, and isolated by HPLC-purification.

Thus, 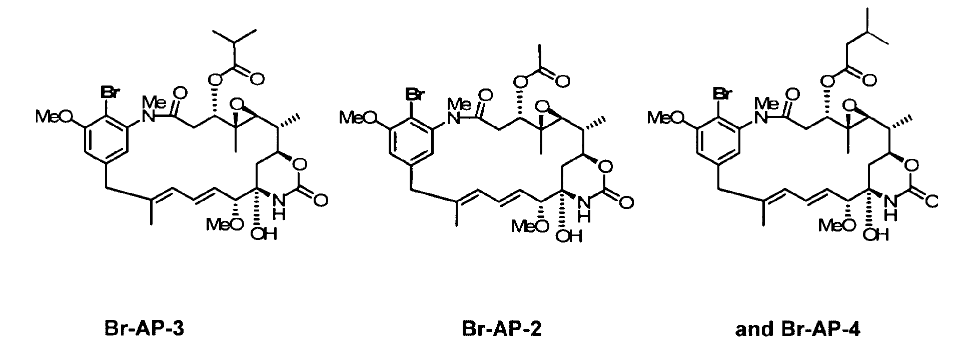 were produced by mutant HGF073. The yield of Br-AP-3 was 50 mg/L culture.

### Production of Ansamitocin derivatives by mutasynthesis

The development of natural product based drugs is often hampered by their structural complexity. This fact precludes facile total synthetic access to analogues or the development of natural product libraries. Therefore, semisynthetic as well as biotechnological approaches are commonly pursued in pharmaceutical research and development (F. von Nussbaum, M. Brands, B. Hinzen, S. Weigand, D. Häbich, Angew. Chem. 2006, 118, 5194-5254; Angew. Chem. Int. Ed. 2006, 45, 5072-5129; I. Paterson, E. A. Anderson, Science 2005, 310, 451-453. c) D. J. Newman, G. M. Cragg, K. M. Snader, J. Nat. Prod. 2003, 66, 1022-1037). A very interesting strategy combines chemical semisynthesis with biosynthesis using genetically engineered microorganisms, a technique which occasionally has been termed mutational biosynthesis or in short mutasynthesis (Review: S. Weist, R. D. Süssmuth, Appl. Microbiol. Biotechnol. 2005, 68, 141-150) According to Rinehart (K. L. Rinehart, K. L. Pure Appl. Chem. 1977, 49, 1361-1384) mutasynthesis involves the generation of biosynthetic blocked mutants, feeding to and incorporation of a mutasynthon by the blocked mutants, isolation of the new metabolites which finally are evaluated for their biological activities.

Ansamitocin derivatives can be obtained by mutasynthesis, e.g. by feeding the growing cultures of Mutant HGF073 with the respective mutasynthons, i.e. 3-aminobenzoic acid and SNAC derivatives instead of AHBA, e.g. mutasynthons selected from

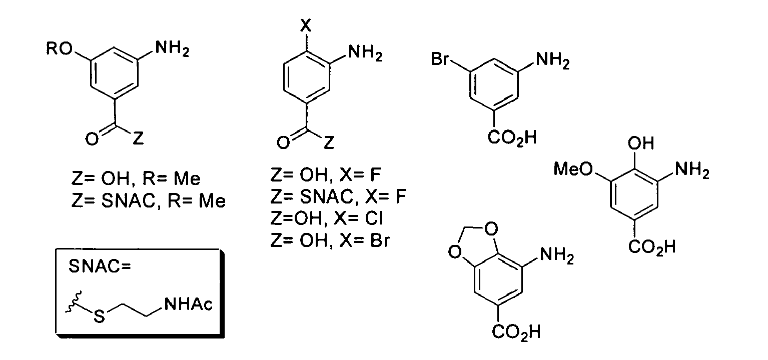

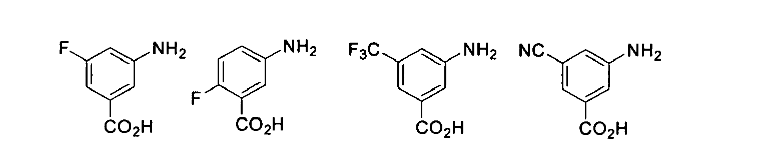

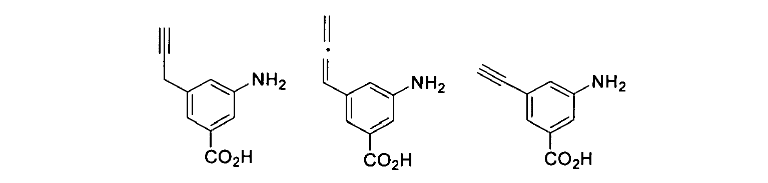

### Example 2:

Mutant HGF073 was cultivated as described above. 3-amino-4-fluorobenzoic acid (37.5 µmol, sterile 25 mM solution in DMSO/H₂O= 1:1) was fed to growing cultures of *Actinosynnema pretiosum* mutant HGF073 in 3 portions of 500 µl after 72, 96 and 120 h incubation in medium K, respectively. The cultures were harvested after 7 days and twice extracted with ethyl acetate (20 ml). The ethyl acetate portions were concentrated in vacuo and the residue was dissolved in methanol (1 ml). Purification was carried out by HPLC (column 250×8 mm (LiCHrospher RP Select B), 10 µm, eluent: methanol/H₂O, gradient 20% methanol to 100% methanol in 30 min, 5 min 100% methanol, flow: 6 ml/min; UV detection at 254 nm). Similarly, 3-amino-4-chlorobenzoic acid, 3-amino-4-bromobenzoic acid, and 3-amino-5-methoxybenzoic acid were fed to growing mutant HGF073 and purified by HPLC. Feeding of the 3-amino-halobenzoic acids resulted in the following demethoxy AP-3 analogues, which were isolated as pure compounds:

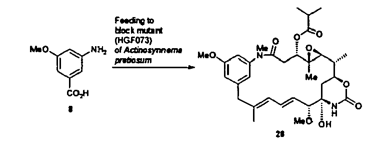

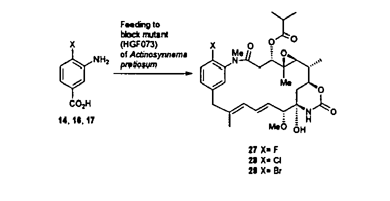

Feeding of 3-amino-5-methoxybenzoic acid to HGF073 and subsequent purification yielded:

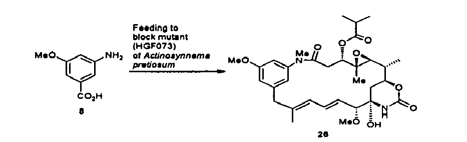

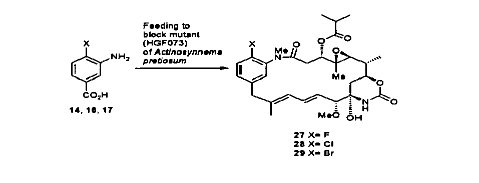

### Chemical derivatization of ansamitocin derivatives

Ansamitocin derivatives having derivatized groups R1 and/or R2 can be prepared using usual chemical reactions and synthesis methods known to a person skilled in the art.

For instance, a compound according to general formula (I) can be prepared by coupling Br-AP-3 or the Br-AP-3 analogue of formula (V) 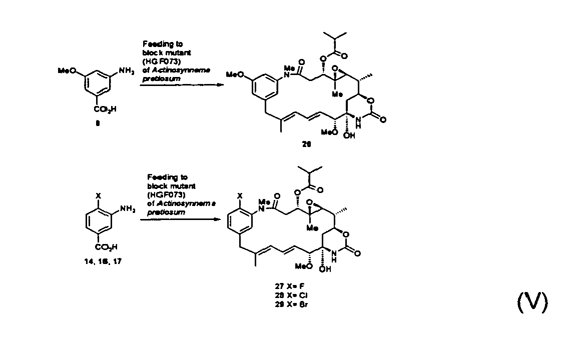 with an organometal compound selected from the group consisting of R⁵-SnR^{e}₃, R⁵-ZnR^{e}, R⁵-AlR^{e}₂, R⁵-Cu, R⁵-In, MgR^{e}, BR^{e}₂, B(OR^{f}₂), BF₃⁻, wherein R⁵ is selected from alkyl, C2-C6 alkenyl, alkynyl, C2-C6 heteroalkyl, aryl, or aralkyl, especially aryl, C1-C6 alkyl, allyl, vinyl, C2-C6 alkinyl, -(CH₂)ₙOH (n= 1-3), -(CH₂)ₙCO₂H (n= 0-2), -(CH₂)ₙNH₂ (n= 0-2), anticalins, antibodies, folic acid, fluorescence dyes, e.g. fluorescein or labels, or molecular probes, e.g. biotin; and each R^{e} is independently selected from methyl, ethyl, butyl or cyclohexyl, and each R^{f} is selected from hydrogen atom, pinacole or cresole.

Preferably, a Stille coupling is carried out, since Stille coupling in contrast to other catalyzed C-C coupling reactions like the Heck-Mizoroki and Suzuki-Miyaura reaction, proceeds under almost neutral conditions.

Concerning the derivatization with antibodies, see e.g. a) R. V. J. Chari, B. A. Martell, J. L. Gross, S. B. Cook, S. A. Shah, W. A. Blättler, S. J. McKenzie, V. S. Goldmacher, Cancer Res. 1992, 52, 127-131; b) K. Okamoto, K. Harada, S. Ikeyama, S. Iwasa, Jpn. J. Cancer Res. 1992, 83, 761-768; c) C. Liu, B. M. Tadayoni, L. A. Bourret, K. M. Mattocks, S. M. Derr, W. C. Widdison, N. L. Kedersha, P. D. Ariniello, V. S. Goldmacher, J. M. Lambert, W. A. Blättler, R. V. J. Chari, Proc. Nat. Acad. Sci. USA 1996, 93, 8618-8623; and d) R. V. Madrigal, B. W. Zilkowski, C. R. Smith Jr., J. Chem. Ecol. 1985, 11, 501-506, and references cited therein.

As for the derivatization with anticalins, see e.g. a) S. Schlehuber, A. Skerra, Expert Opin Biol Ther. 2005, 5, 1453-1462 and b) S. Schlehuber, A. Skerra, Drug Discov Today. 2005, 10, 23-33.

With regard to folic acid, see e.g. a) A. R. Hilgenbrink, P. S. Low, J. Pharm. Sci.; 2005, 94, 2135-46 and b) C. P. Leamon, J. A. Reddy, Adv. Drug Deliv. Rev.; 2004, 56, 1127-1141.

For the attachment of linker groups, see e.g. P. Dubs, R. Stüssi, Helv. Chim. Acta 1976, 59, 1307-1311.

Further, a compound according to general formula (I) can be linked to tumor-associated ligands, e.g. anticalins, antibodies, folic acid, molecular probes or fluorescent dyes by acylation, sulfonation or phosphorylation. A more detailed discussion of the respective procedures can be found, e.g. in T. W. Greene, P. G. M. Wuts, Protective Groups in Organic Synthesis 3rd Edition, John Wiley & Sons, Inc., New York 1999, pp 149.

Example 3: Stille reaction of 19-bromo-20-demethoxy AP-3 (V) with vinylstannane.

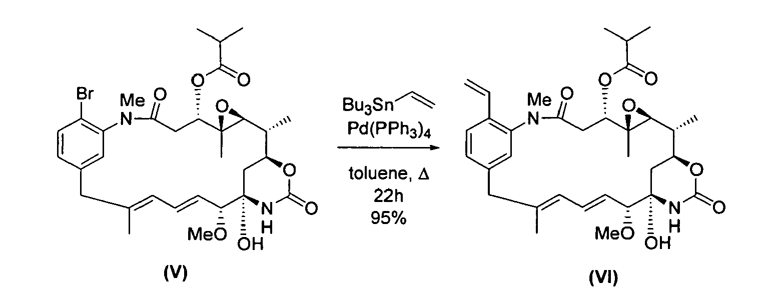

The coupling of tributylvinylstannane with the brominated ansamitocin (V) proceeded in excellent yield. After workup, chromatographic workup purification, 1.3 mg of 20-demethoxy-19-vinyl AP-3 (VI) was isolated as pure material.

### Example 4: Acylation of 19-hydroxy AP-3 (VIII) with an acid chloride.

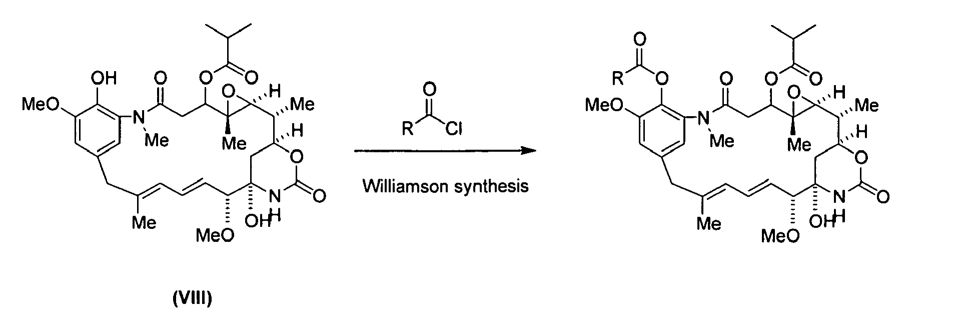

### Example 5: Determination of the antitumor activity of the ansamitocin derivatives according to the present invention.

For the detailed evaluation of their biological profile as anti-cancer agents, cultured human tumor cell lines were treated with the new ansamitocin derivatives. In particular, the 19-fluoro-20-demethoxyansamitocin P3 (F-A), 19-chloro-20-demethoxyansamitocin P3 (Cl-A), 19-bromo-20-demethoxyansamitocin P3 (Br-A), and the 20-demethoxy-19-vinylansamitocin P3 (VI) were tested for their inhibitory effect on cell growth of different cell lines, namely KB-3-1, U-937, PC-3, SK-OV-3, A-498, A-431, and A-549, in comparison to natural biosynthesis product Ansamitocin P-3 (AP-3). Growth inhibition was measured in microtiter plates. 60 µl of serial dilutions of the test compounds were added to aliquots of 120 µl of the suspended cells (50000/ml). After 5 days the growth was determined using a MTT assay, except for U-937, where the WST-1 assay from Roche was employed.

From the data thus obtained (see Table 1 below) it is apparent that all derivatives show very strong activity against leukemia and ovarian cancer cell lines. Lower activity was found against kidney cancer cell lines. Thus, the introduction of different substituents into the ansamitocins results in differential inhibitory action on these cell lines. In general, the 19-fluoro-20-demethoxy-ansamitocin P3 and the 20-demethoxy-19-vinylansamitocin P3 are most active of the compounds tested. The activities of Cl-A and Br-A are generally 3 to 50 times weaker than that of the natural AP-3. The results are shown in Table 1.

**Table 1. Antiproliferative activity IC₅₀ [ng/mL] of AP-3 in comparison to F-A, Cl-A, Br-A and VI**

| Cell line | Origin | **AP-3** | **F-A** | **Cl-A** | **Br-A** | **VI** |
|---|---|---|---|---|---|---|
| KB-3-1 | Cervix carcinoma | 0.11 | 0.18 | 0.35 | 0.3 | 0.4 |
| U-937 | Lymphoma | 0.0035 | 0.01 | 0.035 | 0.03 | 0.05 |
| PC-3 | Prostate carcinoma | 0.035 | 0.035 | 0.12 | 0.1 | 0.1 |
| SK-OV-3 | Ovarian carcinoma | 0.03 | 0.035 | 0.1 | 0.05 | 0.04 |
| A-498 | Kidney carcinoma | 1.1 | 2 | 3 | 4 | 2 |
| A-431 | Epidermoid carcinoma | 0.05 | 0.04 | 0.15 | 0.1 | 0.04 |
| A-549 | Lung carcinoma | 0.1 | 0.11 | 0.4 | 0.3 | 0.14 |

## Claims

1. An ansamitocin derivative of the general formula (I): 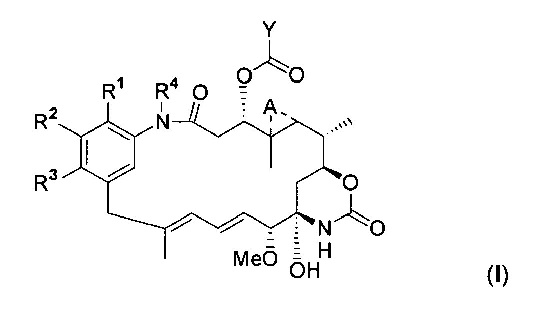 or a pharmacologically acceptable salt, solvate, hydrate or a pharmacologically acceptable formulation thereof, wherein
R¹ is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, aryl, sulfonyl, phosphoryl, anticalins, antibodies, folic acid, fluorescence dyes or labels, or molecular probes, or
R¹ is taken together with R² to form a 5- to 8-membered carbocyclic or heterocyclic ring that is substituted with from 0 to 3 substituents which substituents are each independently selected from halogen atom, hydroxy, nitro, amino, alkoxy, carboxyl, alkyl, alkynyl, alkenyl, or aryl;
R² is a hydrogen atom, a halogen atom, a hydroxy, an amino, a mercapto, an alkyl, an alkenyl, an alkinyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, wherein the alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group can be substituted with from 1 to 3 substituents which substituents are each independently selected from halogen atom, hydroxy, nitro, amino, alkoxy, carboxyl, alkyl, alkynyl, alkenyl, or aryl;
R³ is a hydrogen atom or fluorine atom ;
R⁴ is a hydrogen atom or methyl group;
A represents a bond or an epoxy group;
Y is a methyl, isopropyl, or isobutyl group.

2. The compound according to claim 1, wherein R¹ is a hydrogen atom, halogen atom or hydroxy; or R¹ is taken together with R² to form a 1,3 dioxolen.

3. The compound according to claim 1 or claim 2, wherein R² is a hydrogen atom, halogen atom, cyano, methoxy, trifluoromethyl, propadienyl, ethynyl, propargyl.

4. The compound according to any one of claims 1 to 3, wherein R³ is a hydrogen atom.

5. The compound according to any one of claims 1 to 4, wherein R² is a hydrogen atom or methoxy; and R³ is a hydrogen atom.

6. The compound according to claim 1, wherein R¹ is a hydrogen atom;
R² is a halogen atom, cyano, trifluoromethyl, propadienyl, ethynyl, propargyl; and
R³ is a hydrogen atom.

7. The compound according to any one of claims 1 to 6, wherein R⁴ is a methyl group.

8. The compound according to any one of claims 1 to 7, wherein Y is an isopropyl group.

9. The compound according to any one of claims 1 to 8, wherein A is an epoxy group.

10. The compound according to claim 1, wherein the compound is a compound according to formula (II): 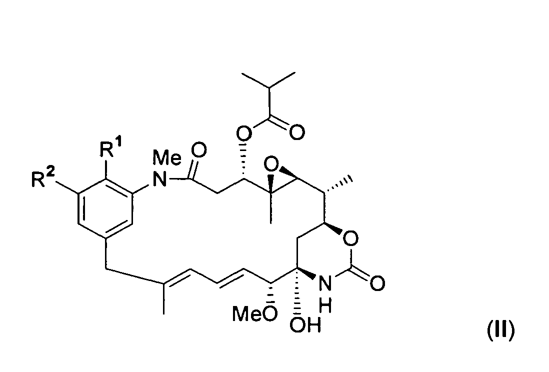 wherein
R¹ is fluorine, chlorine, bromine, iodine or hydroxy; and
R² is hydrogen atom or methoxy.

11. The compound according to claim 1, wherein the compound is a compound according to formula (III): 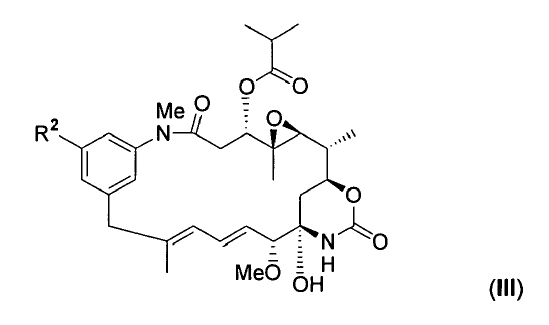 wherein
R² is fluorine, bromine, cyano, trifluoromethyl, propadienyl, ethynyl, or propargyl.

12. The compound according to claim 1, wherein the compound is a compound according to formula (IV):

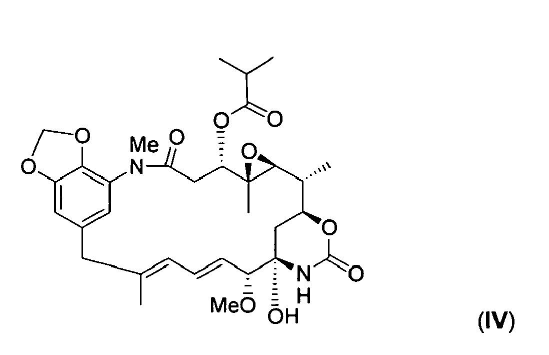

13. A pharmaceutical composition that comprises at least one compound according to any one of claims 1 to 12 and, optionally, a carrier substance and/or an adjuvant.

14. A method for the preparation of a compound of formula (I), wherein R¹ is selected from C1-C6 alkyl, C2-C6 alkenyl; C2-C6 alkynyl, C2-C6 heteroalkyl, aryl, aralkyl, **characterized in that** a compound of formula (V) or formula (VII) 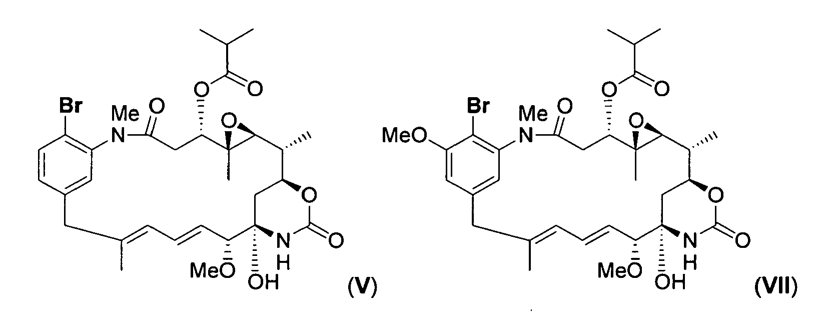 is reacted with an organometal compound selected from the group consisting of R⁵-SnR^{e}₃, R⁵-ZnR^{e}, R⁵-AlR^{e}₂, R⁵-Cu, R⁵-In, MgR^{e}, BR^{e}₂, B(OR^{f}₂), BF₃⁻, wherein R⁵ is selected from alkyl, C2-C6 alkenyl, alkynyl, C2-C6 heteroalkyl, aryl, or aralkyl, especially aryl, C1-C6 alkyl, allyl, vinyl, C2-C6 alkinyl, -(CH₂)ₙOH (n= 1-3), -(CH₂)ₙCO₂H (n= 0-2), -(CH₂)ₙNH₂ (n= 0-2), anticalins, antibodies, folic acid, fluorescence dyes or labels, or molecular probes; and each R^{e} is independently selected from methyl, ethyl, butyl or cyclohexyl, and each R^{f} is selected from hydrogen atom, pinacole or cresole.

15. The method according to claim 14, wherein a Stille coupling is carried out.

16. A method for the preparation of a compound of formula (I), wherein R¹ is alkoxycarbonyl, **characterized in that** a compound of formula (VIII) 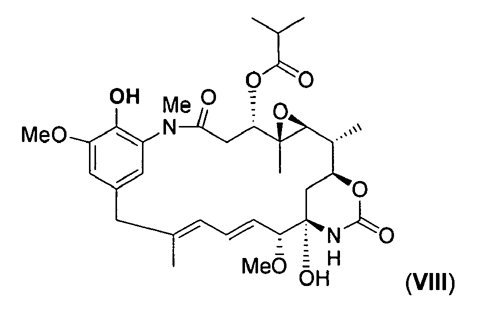 is acylated.

17. A method for the preparation of a compound of formula (I), the method comprising the steps of:
(a) fermenting *Actinosynnema pretiosum* mutant HGF073 in the presence of 3-aminobenzoic acid and SNAC derivatives selected from any of 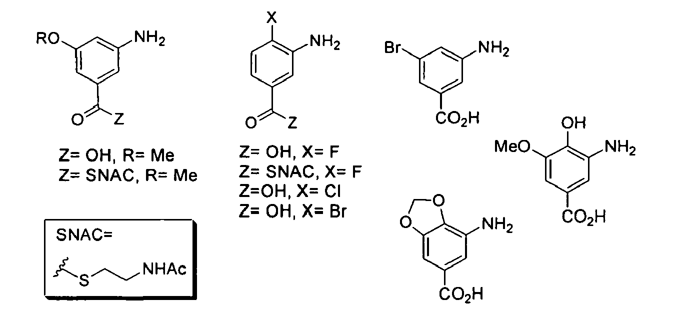 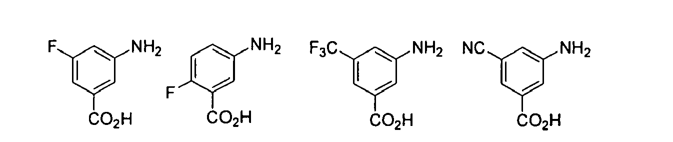 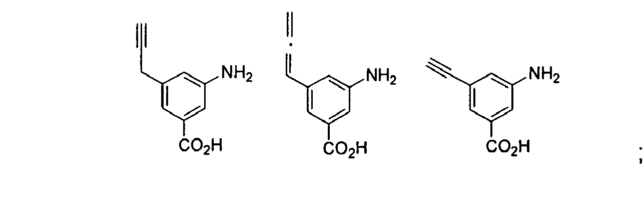 and
(b) separating and retaining the compound from the culture broth.

18. A method for the preparation of a compound of formula (VII) 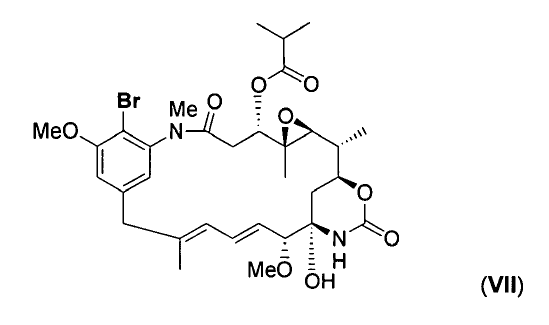 the method comprising the steps of:
(a) fermenting *Actinosynnema pretiosum* mutant HGF073 in the presence of NaBr;
(b) separating and retaining the compound of formula (II) from the culture broth.

19. Compound or pharmaceutical composition according to any one of claims 1 to 12 for use as a pharmaceutical for the treatment of cancer.

20. Compound or pharmaceutical composition according to claim 18, wherein the cancer is leukaemia, ovarian cancer, and kidney cancer.
